# EUROPEAN PATENT APPLICATION

(11) **EP 0 703 295 A1**
(43) Date of publication of application: **27.03.1996**
(21) Application number: 94402141.9
(22) Date of filing: 26.09.1994
(51) Int. Cl.: C12N 15/30, C07K 14/44, A61K 39/008, C12N 15/62, C07K 16/20, C12N 1/11

(54) **Vaccine composition comprising recombinant BCG expressing gp 63 fusion protein**

(71) Applicant: INSTITUT PASTEUR, F-75724 Paris Cédex 15 (FR); INSTITUT PASTEUR DE TUNIS, 1002 Tunis Belvedere (TN)
(72) Inventor: Gicquel, Brigitte, F-75014 Paris (FR); Abdelhak, Sonia, F-75015 Paris (FR); Dellagi, Koussay, Tunis (TN); Louzir, Hechmi, 2070 La Marsa (TN)
(74) Representative: Gutmann, Ernest

(57) **Abstract**

The invention concerns a vaccine composition to induce protective immunity against leishmania. The composition comprises a suitable amount of a recombinant BCG strain transformed with a DNA fragment including a DNA sequence encoding a fusion protein comprising β-lactamase or a fragment thereof and the other membrane protein of leishmania, preferably an outer membrane protein of L.major, more preferably the gp63 protease of L.major.

The vaccine composition is contained in a plasmid such as plasmid pIPS42 deposited at the CNCM on September 26, 1994 under the accession number I-1478.

The invention also concerns a method for inducing protective immunity against L.major to a mammal. The method comprising administering to a mammal the composition described above.

## Description

### Field of the invention

The invention relates to the cloning and expression of the gp63 gene of Leishmania major in BCG. The invention also relates to recombinant vaccine compositions to induce protective immunity against cutaneous leishmaniasis (CL) based on the expression of the gp63 gene in BCG recombinants.

### Background of the invention

The bacille of Calmette and Guérin (BCG) is a particularly attractive vector for the development of recombinant vaccines (Bloom, 1989). It has already been used to immunize more than 2.5 billion people with a very low incidence of serious complications. As its efficacy is unaffected by maternal antibodies, it can be given at or any time after birth. BCG itself is a potent adjuvent. A single inoculum can elicit long-term immunity.

A growing array of integrative and extrachromosomal expression vectors has been developed over recent years, enabling cloning and expression of foreign genes in BCG (Snapper *et al.*, 1988; Ranes *et al*., 1990; Matsuo *et al*., 1990). Various expression cassettes have been described for efficient expression of HIV1 Gag, Env, Pol and Nef, tetanus toxin and β-galactosidase proteins in BCG. Several recombinant strains (rBCG) induce specific cellular and humoral immune responses in mice (Aldovini and Young, 1991; Stover *et al*., 1991; Winter *et al*., 1991; Murray *et al*., 1992). Protective immunity against the Lyme disease elicited by rBCG against challenge with the pathogen has also been reported (Stover *et al*., 1993).

*Leishmania* are obligate intracellular protozoan parasites of macrophages. They cause leishmaniasis, a major infectious disease of considerable public health and economic importance (WHO report, 1984). Leishmaniasis includes a broad spectrum of diseases, ranging from simple cutaneous to visceral leishmaniasis (Chang and Bray, 1985), according to the parasite species and to the host's susceptibility. Upon clinical recovery from most forms of leishmaniasis, there is often effective and long-lasting resistance to recurrent disease. Several studies have shown that cell-mediated immunity rather than humoral antibodies plays a role in the host defense and resistance to reinfection. For the cutaneous form of leishmaniasis, Th1 subsets of CD4+ cells producing γ-IFN are principally host-protective, whereas the Th2 subsets secreting IL-3, IL-4 and IL-5 are disease-promoting (reviewed by Titus *et al*., 1992).

Vaccination against cutaneous leishmaniasis (CL) is feasible in principle, since individuals who develop the disease acquire long-term immunity against reinfection. Deliberate infection with the virulent cutaneous leishmanial parasite has been the basis for vaccination programmes in Israël and in countries of the former USSR (Greenblatt, 1980). However, complications associated with such immunization indicated the need for a killed, attenuated or sub-unit vaccine for CL. Killed *Leishmania* organisms and soluble extracts of promastigotes have been successfully used for immunising animals (Howard *et al*., 1984; Liew *et al*., 1984; Mitchell and Handman, 1983; Scott *et al*., 1987) and humans (Adler and Gunders, 1964; Lainson and Shaw, 1977; Mayrink *et al*., 1985). In addition, administration to mice of several purified leishmanial antigens: lipophosphoglycan (Handman and Mitchell, 1985) and surface glycoproteins, Gp63 (Russell and Alexander, 1988) and Gp46/M2 (Champsi and McMahon-Pratt, 1988), confers significant protection against leishmaniasis. Effective immunization with these sub-unit vaccines requires continuous boosters in the presence of an adjuvant. However the same antigen can induce protection or exacerbation of the disease depending on the route of vaccination (Liew *et al*., 1985 a, b).

Live recombinant vaccines, expressing leishmanial antigens, have been recently investigated as delivery systems for inducing appropriate and long-term immunological responses leading to protection against challenge with *Leishmania* parasites. The different inbred mice strains can be classified as resistant or susceptible to leishmaniasis (reviewed by Blackwell, 1989: Blackwell et al., 1991). Resistant CBA mice orally immunised with the AroA⁻ vaccine strain of *Salmonella thyphimurium* transformed with the full length *L. major gp63* gene preferentially developed a Th1 response that led to protective immunity against infection with *L. mexicana* (Yang *et al*., 1990). Susceptible BALB/c mice immunised with recombinant vaccinia virus expressing GP46/M2 were significantly protected against a challenge with *L.amazonensis* (McMahon-Pratt *et al*., 1993).

A recent report has shown that BCG expression gp63 as a fusion protein with the BCG *hsp60* gene product engendered a protective response against a challenge with *L. mexicana* promastigotes after immunization of CBA and BALB/c mice. This recombinant BCG strain protected resistant CBA mice but not susceptible BALB/c mice against a challenge with *L. major*, although lesion development within this group was less than in untreated and BCG control groups (Connell et al. 1993).

### Summary of the invention

The inventors have cloned and expressed a gene encoding in outer membrane protein of leishmania, particularly the gene encoding a gp63 protease of *Leishmania major* in BCG to develop a recombinant vaccine against zoonotic cutaneous leishmaniasis. Two different expression systems were investigated. The first system consists of *pAN*, a *Mycobacterium paratuberculosis* promoter, which drives expression of ORF2, an open reading frame in IS*900*. This system allows the production of heterologous polypeptides as hybrids with the ORF2 gene product. The second expression system relies on the production of antigenic fragments as fusion proteins with a β-lactamase or a portion thereof, particularly the N terminal region of β-lactamase and more particularly the N-terminal region of *mycobacterium fortuitum* β-lactamase. Both constructs resulted in the production of Gp63 in BCG. The ability of the two recombinant BCG strains to induce protective immunity against a challenge with *L.major* amastigotes was evaluated after vaccination of susceptible (BALB/c), and resistant (C57BL/6) mice. Recombinant BCG producing the Gp63 as a hybrid protein with the N-terminal region of the β-lactamase elicited significant protection against a challenge with *L.major* in BALB/c-immunised mice.

Thus, the invention relates to a recombinant vaccine composition to induce protective immunity against Leishmania and particularly against L. major. The composition comprises a suitable amount of a recombinant BCG strain transformed with a DNA fragment, preferably a plasmid, including a DNA sequence encoding a fusion protein comprising β-lactamase or a fragment thereof and an outer membrane protein of leishmania, particularly of *L. major*, more particularly the gp63 protease of *L. major*.

The invention also includes an immunogenic composition capable of inducing a humoral and cellular response against leishmania, particularly *L. major*, and comprising a fusion protein including β-lactamase or a fragment thereof and a *L. major* outer membrane protein, particularly the gp63 protease of *L. major*. Also within the scope of the invention in a recombinant strain of BCG expressing a fusion protein including β-lactamase or a portion thereof and a gp63 protease of *L. major*. The invention also relates to purified antibodies against a fusion protein comprising a β-lactamase or a fragment thereof and a gp63 protease of *L. major*. The techniques which are used to purify these antibodies are known to those skilled in the art.

The invention also comprises the use of a composition as defined above to modulate the immune response of a mammal to a leishmania antigen in an ongoing leishmania infection.

The invention also relates to a method for inducing protective immunity against L. major in a mammal. The method comprises administering to the mammal a vaccine composition as defined previously. Particularly, the composition is administered either intravenously and comprises between 10⁶ and 5 x 10⁶ CFU of the recombinant BCG, defined above or subcutaneously and comprises between 10⁷ and 2 x 10⁷ CFU of the recombinant BCG defined above.

The invention also relates to a recombinant strain of BCG expression a fusion protein including β-lactamase or a portion thereof and a gp63 protease of *L. major*. Particularly, the invention relates to a recombinant BCG strain comprising plasmid pIPS42 deposited at the Collection Nationale de Cultures de Microorganismes on September 26, 1994 under accession number I-1478 and identified as MYC1155.

In the drawings,
**Figure 1** represents the construction of recombinant BCG vectors. Only plasmid inserts are shown.
**Figure 2** represents the Western blot analysis of recombinant BCG clones with an anti-Gp63 monoclonal antibody.
   **Lane 1:** Total protein extract from BCG1173P2.
   **Lane 2:** Total protein extract from BCG clone harbouring pIPS42
   **Lane 3:** Total protein extract from BCG clone harbouringpIPS11.
   **Lane 4:** *L. major* promastigote membrane extract.
**Figure 3** represents the evaluation of protection against *L.major* infection in BALB/c (**A** and **B**) and C57BL/6 (**C** and **D**) mice immunised subcutaneously (**A** and **C**) or intravenously (**B** and **D**) with classical (1173P2) or with recombinant BCG. Mean footpad swelling for each mouse group.is indicated * indicates the number of mice that lost their footpad. control 1173P2 IPS42 IPS11
**Figure 4** represents the proliferative responses to soluble leishmanial antigens (SLA) of popliteal lymph node cells of BALB/c (**1**) and C57BL/6 (**2**) mice. Each point represents the proliferative response in Æ cpm for a single mouse receiving either recombinant (IPS42) (**A**) or non-recombinant BCG (**B**) or ofthe control unimmunised group (**C**), challenged with *L. major* amastigotes.

### Bacterial strains, plasmids, parasites and culture conditions

The bacterial strains, plasmids and parasites used in this study are listed in table 1. *E. coli* strains were grown in L-broth supplemented with appropriate antibiotics and were transformed as previously described (Sambrook *et al*., 1989). Mycobacterial strains were transformed by electroporation as described previously (Ranes *et al*., 1990). Recombinant BCG clones were first cultivated on Lowenstein-Jensen medium containing 10µg/ml Kanamycin, then transferred onto Sauton medium for classical vaccine preparation or grown as dispersed culture (Gheorghiu *et al*., 1988). *L. major* strain TN435 was cultured in RPMI 1640 supplemented with 10% inactivated foetal calf serum (Hyclone Laboratories, Utah, USA) (Jaffe *et al*., 1984). *L. major* amastigotes were obtained by passaging strain PSA.18.SBZ isolated from *Psammomys obesus* from the region of Sidi Bou Zid (Tunisia) and passaged *in vivo* 5 times in BALB/c mice.

### Cloning and expression of gp63 in BCG

Two different expression systems were used to drive the expression of *gp63* in BCG. The first system allows the production of heterologous proteins in *Mycobacteria* as fusions with the N-terminus of the protein encoded by ORF2, an open reading frame of 382 codons which belongs to the insertion sequence IS*900* isolated from *Mycobacterium paratuberculosis*. Expression of the fusion is driven by *pAN*, a promoter located upstream ORF2 (Murray *et al*., 1992). Previous studies have shown that analogous fusions of the *lacZ* gene to the ORF2 sequence result in the production of fusion proteins with β-galactosidase activity in BCG (Murra*y et al*., 1992). This recombinant BCG elicited T cell proliferative responses and anti-β-galactosidase antibody production in immunised mice.

The second expression system was recently developed for the production and export of heterologous proteins in *Mycobacteria* (Timm *et al*., 1994 incorporated by reference). This system relies on the production of antigenic fragments as fusions with the N-terminal region of β-lactamase (BlaF). Expression is driven by a mutant of β-lactamase gene promoter (*pblaF**) isolated from a *M. fortuitum* strain overproducing β-lactamase.

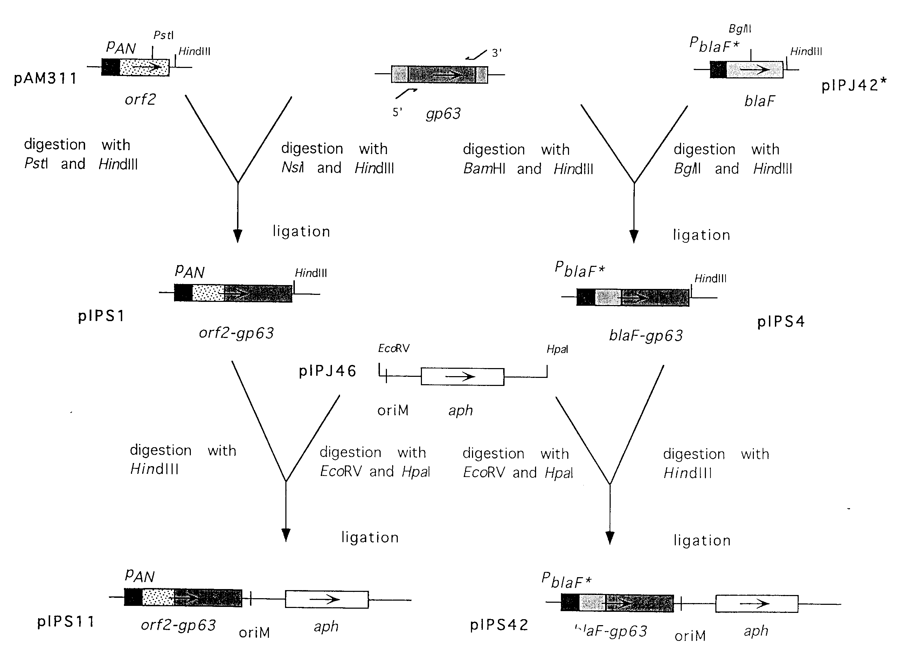

The Gp63 protein precursor of *L. major* is encoded by an open reading frame of 1.8 kb of which 1.5 kb encodes the mature form of Gp63 (Button and McMaster, 1988). The DNA fragment coding for the mature protein was synthesised *in vitro* by PCR amplification from genomic DNA of *L.major* strain TN435,(provided by R. Ben Ismaïl (Institut Pasteur de Tunis) (Ben Ismaïl *et al*., 1986). Translational initiation and termination codons, and restriction sites for the subsequent cloning were added (Fig. 1).

The sequences of the 5' and 3' primers are
5'-GCGGGATCCTTATGC**AT****G**TGCGCGACGTGAACTGGGGC-3'
identified as Seq. ID No. 1 (the BamHI and NsiI sites are underlined) and
5'-CCGAATTCAAGCTT**CTA**CGCCGTGTTGCCGCCGTCCTT-3'
identified as Seq. ID No. 2 (the HindIII site is underlined) an ATG start codon and a stop codon were included (in bold). The gp63 gene obtained by PCR was then cloned in plasmids pIPJ42* and pAM311 as shown in figure 1. Plasmid pIPJ42* is a pUC18 vector harbouring at the BamHI site the β-lactamase gene: *bla F* with the regulatory region of *blaF**, which overproduces β-lactamase (Timm *et al*., 1994). Plasmid pAM311 is a pUC18 derivative containing a 1.6 kb fragment from *M. paratuberculosis* harbouring the *pAN* promoter and ORF2 (Murray *et al*., 1992). pIPJ46 is a pSL1180 derivative harbouring the origin of replication of the *M. fortuitum* plasmid pAL5000 (Ranes *et al*., 1990) and the kanamycin-resistance gene from Tn*903* (Kanamycin Resistance Gen-Block, Pharmacia).

The resulting *E. coli*/ mycobacteria shuttle plasmids were named respectively pIPS11 and pIPS42. These plasmids were introduced into *M. smegmatis* and a BCG strain deposited at CNCM on April 24, 1978 under accession number I-059 by electroporation as previously described (Winter et al., 1991).

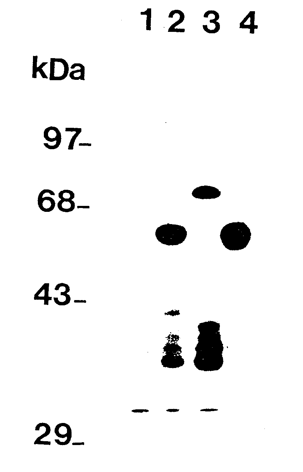

The production of the fused Gp63 proteins in BCG was assessed by Western blot analysis of total protein extracts using an anti-Gp63 monoclonal antibody (Button et al., 1991, kindly provided by W.R. McMaster), (Fig. 2).

Proteins were separated on 0.1% SDS-10% polyacrylamide gels according to (Laemmli, 1970). After electroblotting, the membranes were incubated with the monoclonal antibody. The membranes were washed and incubated with horseradish peroxidase-conjugated sheep anti-mouse second antibody and developed with the ECL chemiluminescent substrate according to the manufacturer's instructions (Amersham Corp.).

The antibody bound two major products in recombinant BCG strains: 73 kDa and 63 kDa polypeptides corresponding to the fusion products between the N-terminal part of the polypeptide encoded by ORF2 or of β-lactamase respectively and Gp63. The amount of the β-lactamase-Gp63 hybrid was consistently higher than that of the hybrid of Gp63 with the polypeptide encoded by ORF2 (Fig. 2 and data not shown). This is in accordance with studies showing that *lacZ* expression in BCG from *pblaF** resulted in a β-galactosidase activity, in transformed *BCG* which was 6 times higher than that from *pAN*. The β-lactamase-Gp63 fusion protein was found only in the total extract of the recombinant BCG cells and not in the culture.

### Protection against infection with L. major:

The amount of recombinant BCG to be injected as well as its schedule of administration vary depending on the individual to be vaccinated and upon the chosen route of administration.

Generally, between 1 and 2 injections at 3 to 4 weeks interval of 10⁶ to 5 x 10 ⁶ CFU of the recombinant BCG of the invention is suitable for intravenal vaccination. As for subcutaneous vaccine, between 1 and 2 injections at 3 to 4 weeks interval of 10⁷ to 2 x 10⁷ CFU of the recombinant BCG are required.

It is to be noted that adjuvents can be added to the vaccine composition to enhance the level of immunological response of the immunized mammal.

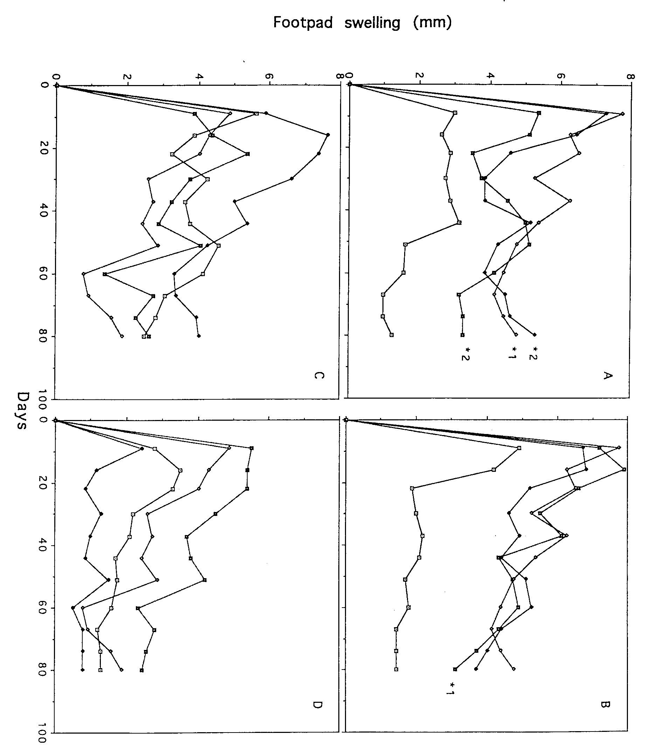

By way of example, four groups of 8 to 10 BALB/c or C57BL/6 mice were immunized twice at one month interval with 10⁶ cfu iv or 10⁷ sc of the BCG vaccine strain 1173P2, recombinant BCG strains IPS42 and IPS11, or no vaccine (naive mice). One month after the booster injection, mice were challenged in one hind footpad with 2.10⁶ amastigotes isolated from the footpad of an infected mouse as follows: the foot was ground with a cell dissociation sieve-tissue grinder kit (Sigma). Amastigotes were counted and resuspended in PBS at a concentration of 4.10⁷/ml.The development of cutaneous lesions was followed for 11 weeks after infection by measuring footpad swelling (Fig. 3).

The footpad swelling was estimated by measurement of the foot circumference and by subtracting the foot circumference on the day of challenge.

The *L. major* strain TN435 caused a progressive disease in BALB/c mice, but a self resolving disease in C57BL/6 mice (figure 3). The ulcerative lesions induced in BALB/c mice by *L. major* strain TN435 were smaller than those caused by other strains described in the literature (8 to 15 mm). The lesions reached a maximum size in 2 to 4 weeks and then stabilized at an average diameter of 4 to 6 mm.

BALB/c mice inoculated with BCG producing the Gp63-BlaF fusion protein (IPS42) had significantly smaller lesions than controls. Recombinant BCG administered by either route restricted lesion development. However, iv administration was associated with a more rapid regression of the lesion as its diameter regressed significantly from day 22 (figure 3). Mouse groups immunised, by sc or iv route, with the non recombinant BCG (1173P2) vaccine or with the recombinant BCG producing the Gp63-ORF2 fusion (IPS11), developed ulcerous lesions similar in size to those in the unimmunised control group.

C57BL/6 mice which did not receive BCG developed only a footpad swelling that regressed significantly 6 weeks after challenge with *L. major* amastigotes, as has been reported for other mouse strains resistant to infection with *L. major*. The C57BL/6 mice, immunised subcutaneously or intravenously with BCG or with either recombinant BCG-*gp63*, developed footpad swelling similar to that in the control group. Accelerated healing in C57BL/6 mice naturally resistant to *L. major* infection, was observed only after iv immunisation. This is consistent with previous reports describing a protective effect of BCG against *L. major* infection in mice (Fortier *et al*., 1987; Connell *et al*., 1993). In contrast to the control group, the footpad swelling of the immunised C57BL/6 mice did not regress completely 8 weeks after challenge. These lesions appeared different from those induced in susceptible BLAB/c mice, since they were not ulcerated and were found free of parasite at day 51 and at the end of the study. An histological exam of the foodpad tumefaction which was performed in two mice of these groups, showed a faint interstitiel oedema and a subcutaneous infiltrate of lymphocytes with few histiocytic cells. No leishman bodies were detected.

### Evaluation of parasite load and cellular immune responses

The best protection against *L.major* infection was thus obtained in BALB/c mice with recombinant BCG expressing Gp63 as a fusion protein with N-terminal region of β-lactamase (IPS42). Since the subcutaneous route of immunization appeared promising, we evaluated the cellular immune responses and the number of parasites at the site of challenge in mice injected subcutaneously during the protocol described above. Three groups of 8 BALB/c and C57BL/6 mice received subcutaneously either IPS42 or non recombinant BCG or did not receive any vaccine. The mice were then challenged with *L. major* and sacrificed 120 days later. The footpad of each mouse was ground in 2 ml RPMI medium and 20µl of tissue homogenate was analysed. The presence of one or more parasite per ten 40-times magnification fields was scored as positive. In BALB/c mice immunised subcutaneously with IPS42, parasites were detected in 2 of 8 mice. Five of 8 mice immunised with BCG and 8/8 unimmunised mice were infected. In C57BL/6 mice no parasites were detected in any group studied.

The proliferative responses of lymph node cells were also investigated in the different groups of mice listed above.

At day 120 after infection with *L. major*, mice were killed, and popliteal lymph nodes draining the cutaneous lesion removed. Mononuclear cells were plated at 4.10⁵ cells per well in 96-well tissue culture plates in 200µl of RPMI 1640 (Flow Laboratories, Puteaux, France) supplemented with 5% heat-inactivated foetal calf serum (Hyclone Laboratories, Utah, USA) penicillin (100 U/ml), streptomycin (50µg /ml), and 5.10⁻⁵M β 2-mercaptoethanol. Promastigotes from *L. major* strain TN435 were sonicated and centrifuged at 47000g and the supernatant containing soluble leishmanial antigens (SLA) (Sacks *et al*., 1987), was used as stimulating antigen at the final concentration of 20µg/ml. Cultures were incubated at 37°C with 5% CO₂, and at day 5, cells were pulsed with 0.5µCi [³H] thymidine (Amersham, UK) for 18 hours. Cells were then harvested and counted with a β scintillation counter. The results are expressed as mean counts per minute (cpm) of triplicate cultures and Æ-cpm (mean counts of antigen stimulated mononuclear cells - mean counts of unstimulated mononuclear cells).

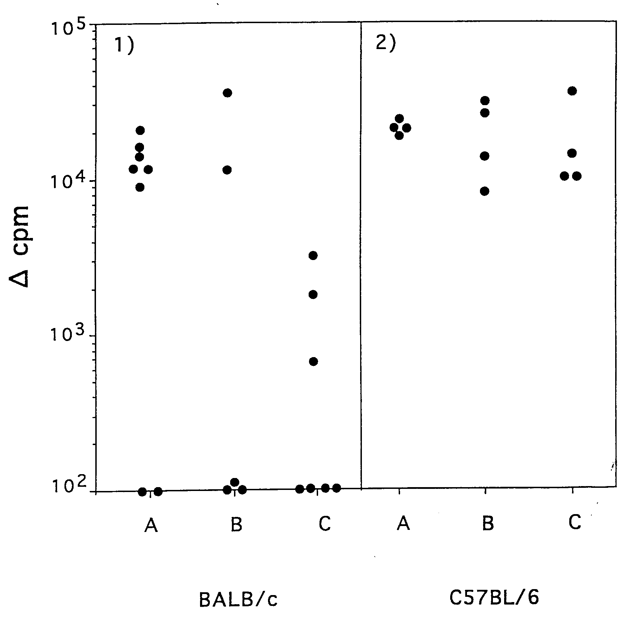

Cells from 6 of 8 mice immunised with IPS42 proliferated vigorously in response to SLA (Fig. 4). In this group the unresponsive mice were those that had persistent cutaneous lesions. Two of 6 BALB/c mice immunised with BCG developed a lymphoproliferative response to SLA. The responsive mice had no parasites at the end of the study. All 6 BALB/c mice of the control group had low proliferative responses. In C57BL/6 mice strong proliferation responses specific to SLA were found in all the groups tested.

Hence, BALB/c mice were protected against *L. major* infection by a recombinant BCG expressing Gp63 as a fusion protein with β-lactamase. Surprisingly the same antigen expressed as a fusion protein with the IS*900* ORF2 gene product failed to induce protective immunity. This might be due to the level of expression of *gp63*, as differences in the expression level of heterologous protein between the two systems have been observed *in vitro* (a factor of 6 in *pblaF** versus *pAN* driven expression).

The ability of the *gp63*-recombinant BCG strain to induce protection against *L. major* infection seems to be independent of the route of vaccination: only slight differences in the protective responses were observed between the i.v. or s.c. inoculation routes.

The reduction of lesion size in BALB/c mice immunised with recombinant BCG IPS42 correlated with the clearance of parasites at the site of challenge. C57BL/6 mice are resistant to leishmaniasis and develop protective proliferative responses during infection. There is no proliferative response during acute disease in BALB/c mice. Acellular response was induced in BALB/c mice where a protective effect was observed.

The present invention shows that immunization with recombinant BCG harbouring *gp63* on a replicative plasmid can elicit appropriate immunological responses resulting in protection against infection. This study shows that recombinant BCG expressing foreign antigens can provide the basis for an effective vaccine against various pathogens.

### References:

Adler, S. and Gunders, A.E. (1964) Immunity of *Leishmania mexicana* following spontaneous recovery from oriental sore. *Trans R Soc Trop Med Hyg* **58:** 274-277.
Aldovini, A. and Young, R.A. (1991) Humoral and cell-mediated immune responses to live recombinant BCG-HIV vaccines. *Nature* **351:** 479-482.
Ben Ismaïl, R., Gradoni, L., Gramiccia, M., Behini, S. and Ben Rachid, M.S. (1986) Epidemic cutaneous leishmaniasis in Tunisia. Biochemical characterization of parasites. *Trans. R Soc Trop Med Hyg* **80:** 669.
Blackwell, J.M. (1989) The macrophage resistance gene *Esh/Ity/Bcg*. 27th forum in immunology. *Res. Immunol.* **40:** 767-826.
Blackwell, J.M., Roach, T.I.A., Atkinson, S.E., Ajioka, J.W., Barton, C.H. and Shaw, M.A. (1991) Genetic regulation of macrophage priming activation: the *Lsh* gene story *Immunol. lett*. 30: 241-248.
Bloom, B.R. (1989) Vaccines for the third world. *Nature* **342:** 115-120.
Bordier, C. (1987) The promastigote surface protease of Leishmania. *Parasit Today* **3:** 151-153.
Button, L.L. and McMaster, W.R. (1988) Molecular cloning of the major surface antigen of Leishmania. *J Exp Med* **167**: 724-729.
Button, L.L., Reiner, N.E. and McMaster, W.R. (1991) Modification of gp63 genes from diverse species of *Leishmania* for expression of recombinant protein at high levels in *Esherichia coli. Mol Biochem Parasit* **44:** 213-224.
Casadaban, M.J. and Cohen, S.N. (1980) Analysis of gene control signals by DNA fusion and cloning in *Escherichia coli. J Mol Biol* **138:** 179-207.
Champsi, J. and McMahon-Pratt, D. (1988) Membrane glycoprotein M-2 protects against *Leishmania amazonensis* infection. *Inf Immun* **56:** 3272-3279.
Chang, K.P. and Bray, R.S. (eds) (1985). In Human parasitic diseases: The leishmaniasis. Amsterdam: Elsevier Science Publishers, pp. 1-481.
Chaudhuri, G., Chaudhuri, M., Pan, A. and Chang, K.P. (1989) Surface acid proteinase (gp63) of *Leishmania mexicana*: a metalloenzyme capable of protecting liposome-encapsulated proteins from the phagolysosomal degradation by macrophage. *J Biol Chem* **264:** 7483-7489.
Connell, N.D., Medina-Acosta, E., McMaster, W.R., Bloom, B.R. and Russell, D.G. (1993) Effective immunization against cutaneous leishmaniasis with recombinant bacille Calmette-Guérin expressing the *Leishmania* surface proteinase gp63. *Proc Natl Acad Sci USA* **90:** 11473-11477.
Fortier, A.H., Mock, B.A., Meltzer, M.S. and Nacy, C.A. (1987) Mycobacteriuin bovis BCG-induced protection against cutaneous- and systemic *Leishmania major* infections of mice. *Infect Immun* **55:** 1707-1714. Gheorghiu, M., Lagrange, P. and Fillastre, C. (1988) The stability and immunogenicity of a dispersed grown freeze derived Pasteur BCG vaccine. *J Biol Stand* **16:** 15-26.
Greenblatt, C.L. (1980) The present and the future of vaccination for cutaneous leishmaniasis. In *New development with human and veterinary vaccines*. Mezhari, A., Hertman, I., Klinger, M.A. and Kohn, A. (eds). New York: Alan R. Liss, Inc., pp. 259-285.
Handman, E. and Mitchell, G.F. (1985) Immunisation with *Leishmania* receptor for macrophages protects mice against cutaneous leishmaniasis. *Proc natl Acad Sci USA* **82:** 5910-5914.
Howard, J.G., Liew, F.Y., Hale, C. and Nicklin, S. (1984) Prophylactic immunization against experimental leishmaniasis. II. Further characterization of the protective immunity against fatal *Leishmania tropica* infection induced by irradiated promastigotes. *J Immunol* **134:** 450-455.
Jaffe, C.L., Grimaldi, G. and McMahon-Pratt, D. (1984) The cultivation and the cloning of *Leishmania*. In *Genes and antigens of parasites. A laboratory manual 2nd Ed. The proceedings of a course sponsored by UNDP World Bank WHO*. Morel, C.M. (eds). Rio de Janeiro, Brazil: pp. 47.
Laemmli, U.K. (1970) Cleavage of structural proteins during the assembly of the head of bacteriophage T4. *Nature* **227:** 680-685.
Lainson, R. and Shaw, J.J. (1977) Leishmaniasis in Brazil. XII. Observations on cross-immunity in monkeys and man infected with *Leishmania mexicana mexicana*, *L. m. amazonensis*, *L. braziliensis braziliensis*, *L. b. panamensis*. *Am J Trop Med Hyg* **80:** 29-35.
Liew, F.Y., Howard, J.G. and Hale, C. (1984) Prophylactic immunization against experimental leishmaniasis. III. Protection against fatal *Leishmania tropica* infection induced by irradiated promastigotes involves Lyt-1⁺2⁻ cells that do not mediate cutaneous DTH. *J Immunol* **134:** 456-461.
Liew, F.Y., Hale, C. and Howard, J.G. (1985a) Prophylactic immunization against experimental leishmaniasis IV. Subcutaneous immunization prevents the induction of protective immunity against fatal *Leishmania major* infection. *J Immunol* **135:** 2095-2101.
Liew, F.Y., Singleton, A., Cillari, E. and Howard, J.G. (1985b) Prophylactic immunization against experimental leishmaniasis V. Mechanism of the anti-protective blocking effect induced by subcutaneous immunization against *Leishmania major* infection. *J Immunol* **135:** 2102-2107.
Matsuo, K., Yamaguchi, R., Yamasaki, A., Tasaka, K., Terasaka, M., Totsuka, K., Kobayashi, H., Yukitake, H. and Yamada, T. (1990) Establishment of a foreign secretion system in mycobacteria. *Infect Immun* **58:** 4049-4054.
Mayrink, W.P., Williams, P., Da costa, C.A., Magalhaes, P.A., Melo, M.N., Dias, M., Oliveira Lima, A., Michalick, M.S.M., Ferrura Carvalho, E., Barros, G.C., Sessa, P.A. and De Alencar, J.T.A. (1985) An experimental vaccine against American dermal leishmaniasis: experience in the State of Espirito Santo. *Brazil Ann Trop Med Parsit* **79:** 259-269.
McMahon-Pratt, D., Rodriguez, D., Rodriguez, J.R., Zhang, Y., Manson, K., Bergman, C., Rivas, L., Rodrigez, J.F., Lohman, K.L., Ruddle, N.H. and Esteban, M. (1993) Recombinant vaccinia viruses expressing gp46/M-2 protect against *Leishmania* infection. *Inf Immun* **61:** 3351-3359.
Mitchell, G.F. and Handman, E. (1983) Leishmania tropica major in mice: vaccination against cutaneous leishmaniasis in mice of high genetic susceptibility. *Aust, J Exp Biol Med Sci* **61:** 11-25.
Murray, A., Winter, N., Lagranderie, M., Hill, D.F., Rauzier, J., Timm, J., Leclerc, C., Moriarty, K.M., Georghiu, M. and Gicquel, B. (1992) Expression of *Escherichia coli* β-lactamase in *Mycobacterium bovis* BCG using an expression system isolated from *Mycobacterium paratuberculosis* which induced humoral and cellular immune responses. *Molec Microbiol* **6:** 3331-3342.
Ranes, M.G., Rauzier J., Lagranderie M., M., G. and B., G. (1990) Functional analysis of pAL5000, a plasmid from *Mycobacterium fortuitum*: Construction of a "Mini" mycobacterium-*Escherichia coli* shuttle vector. *J Bacteriol* **172:** 2793-2797.
Russell, D.G. and Alexander, J. (1988) Effective immunization against cutaneous leishmaniasis with defined membrane-antigens reconstituted into liposomes. *J Immunol* **140:** 1274-1279.
Russell, D.G. and Wilhelm, H. (1986) The involvment of the major surface glycoprotein (gp63) of leishmania promastigotes in attachment to macrophages. *J Immunol* **136:** 2613-2620.
Sacks, D.L., Suman, L.L., Shrivastava, S.N., Blackwell, J. and Neva, F.A. (1987) An analysis of T cell responsiveness in indian Kala-Azar. *J Immunol* **138:** 908-913.
Sambrook, J., Fritsch, E.F. and Maniatis, T. (1989) *Molecular Cloning a laboratory manual*. New York: Cold Spring Harbor.
Scott, P.E., Pearce, E., Natovitz, P. and Sher, A. (1987) Vaccination against cutaneous leishmaniasis in a murine model. I. Induction of protective immunity with a soluble extract of promastigotes. *J Immunol* **139:** 221-227.
Snapper, S.B., Lugosi, L., Jekkel, A., Melton, R.E., Kieser, T., Bloom, B.R. and Jacobs Jr., W.R. (1988) Lysogeny and transformation in mycobacteria: stable expression of foreign genes. *Proc Natl Acad Sci USA* **85:** 6987-6991.
Stover, C.K., de la Cruz, V.F., Feurst, T.R., Burlein, J.E., Benson, L.A., Bennett, L.T., Bansal, G.P., Young, J.F., Lee, M.H., Hatfull, G.F., Snapper, S.B., Barletta, R.G., Jacobs, W.R. and Bloom, B.R. (1991) New use of BCG for recombinant vaccines. **351:** 456-460.
Stover, C.K., Bansal, G.P., Hanson, M.S., Burlein, J.E., Palaszynski, S.R., Young, J.F., Koenig, S., Young, D.B., Sadziene, A. and Barbour, A.G. (1993) Protective immunity elicited by recombinant Bacille Calmette-Guerin (BCG) expressing outer surface protein A (OspA) lipoprotein: a candidate Lyme disease vaccine. *J Exp Med* **178:** 197-209.
Timm, J., M.G., P., Duez, C., Trias, J., Orefici, G., Fattorini, L., Amicosante, G., Oratore, A., Joris, B., Frère, J.M., Pugsley, A.P. and Gicquel, B. (1994) Transcription and expression analysis, using *lacZ* and *PhoA* gene fusions, of *Mycobacterium fortuitum* β-lactamase genes cloned from a natural isolate and a high-level β-lactamase producer. *Mol Microbiol* **12:** 491-504.
Titus, R.G., Theodos, C.M., Kimsey, P.B., Shankar, A., Hall, L., McGurn, M. and Povinelli, L. (1992) Role of T cells in immunity to the intracellular pathogen, *Leishmania major*. In *Subcellular Biochemistry*. Avila, J.L. and Harris, J.R. (eds). New York: Plenum Press, pp. 99-129.
Webb, J.R., Button, L.L. and McMaster, W.R. (1991) Heterogeneity of the genes encoding the major surface glycoprotein of *Leishmania donovani. Mol Biochem Parasitol* **48:** 173-184.
WHO Report (1984) The leishmaniases. *In Report of a WHO Expert Commitee. Technical Report Series*. (eds). pp. 140.
Winter, N., Lagranderie, M., Rauzier, J., Timm, J., Leclerc, C., Guy, B., Kieny, M.P., Gherghiu, M. and B., G. (1991) Expression of heterologous genes in *Mycobacterium bovis* BCG: induction of a cellular response against HIV- 1 Nef protein. *Gene* **109:** 47-54.
Yang, D.M., Fairweather, N., Button, L.L., McMaster, W.R., Kahl, L.P. and Liew, F.Y. (1990) Oral *Salmonella typhimurium* (AroA-) vaccine expressing a major leishmanial surface protein (gp63) preferentially induces T helper 1 cells and protective immunity against leishmaniasis. *J Immunol* **145:** 2281-2285.

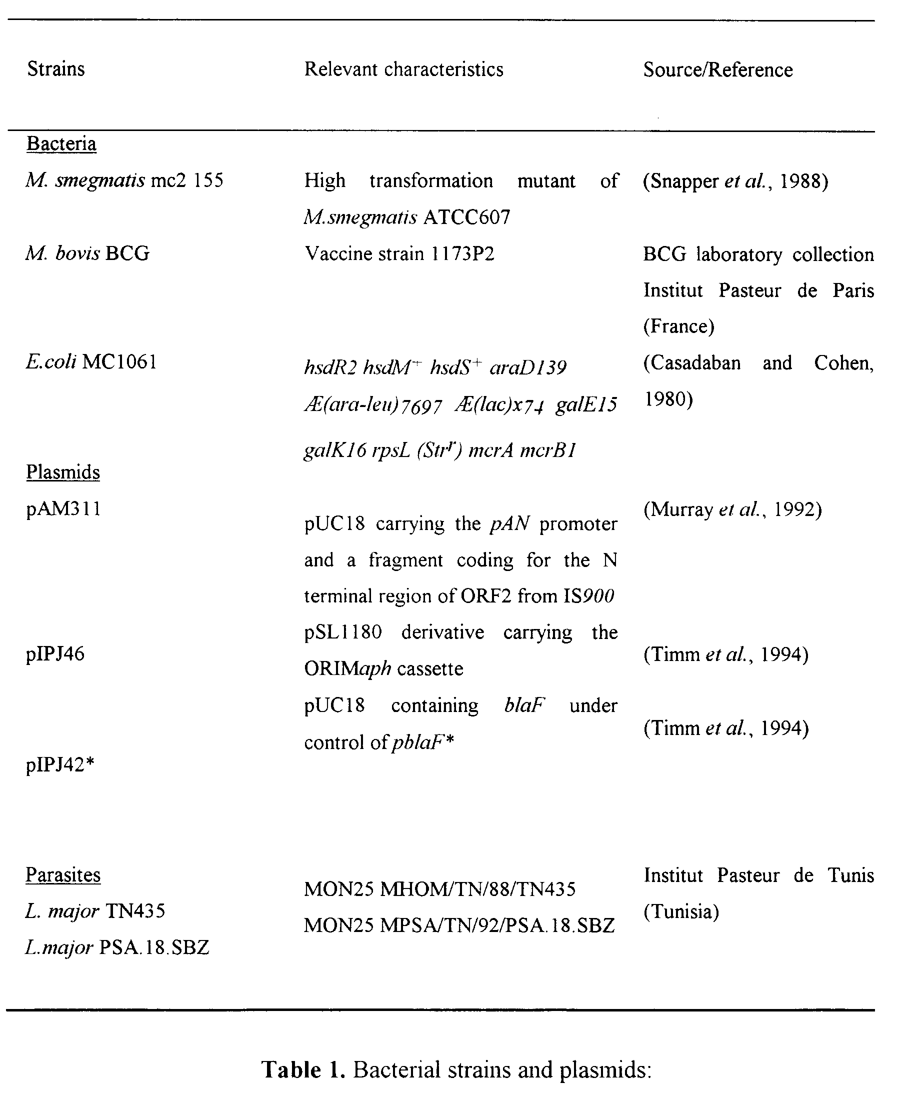

## Claims

1. A vaccine composition to induce protective immunity against leishmania, said composition comprising a suitable amount of a recombinant BCG strain transformed with a DNA fragment including a DNA sequence encoding a fusion protein comprising β-lactamase or a fragment thereof and the outer membrane protein of leishmania.

2. A vaccine composition according to claim 1, wherein said vaccine composition induces protective immunity against L. major.

3. A vaccine composition according to claim 1 or 2, wherein said major outer-membrane protein of leishmania is an outer membrane protein of L. major.

4. A vaccine composition according to claim 3, wherein said outer membrane protein of L. major is the gp63 protease of L. major.

5. A vaccine composition according to claims 1 to 4, wherein said DNA sequence is contained in a plasmid.

6. A vaccine composition according to claim 5, wherein said plasmid is plasmid pIPS42 deposited at the CNCM on September 26, 1994 under the accession number I-1478..

7. A vaccine composition according to any of claims 1 to 6, wherein the amount of recombinant BCG ranges between 10⁶ and 2 x 10⁷ CFU.

8. A vaccine composition according to any of claims 1 to 7, which further comprises a suitable amount of an immunological adjuvant.

9. An immunogenic composition capable of inducing a humoral and cellular response against Leishmania, said composition comprising a fusion protein including β-lactamase or a fragment thereof and the L. major outer membrane protein.

10. An immunogenic composition according to claim 9, wherein said fusion protein includes β-lactamase or a fragment thereof and the gp63 protease of L. major.

11. A method for inducing protective immunity against L.major to a mammal, said method comprising administering to said mammal a composition according to any of claims 1 to 10.

12. A method according to claim 11, wherein when said composition comprises a recombinant BCG strain and is administered intravenously, said composition comprises between 10⁶ and 5 x 10⁶ CFU of said recombinant BCG.

13. A method according to claim 11, wherein when said composition comprises a recombinant BCG strain and is administered subcutaneously, said composition comprises between 10⁷ and 2 x 10⁷ CFU of said recombinant BCG.

14. A method according to any of claims 11 to 13, wherein said composition is administered twice at an interval range between 3 and 4 weeks.

15. Use of a composition according to any of claims 1 to 10 to modulate the immune response of a mammal to a leishmania antigen in an ongoing leishmania infection.

16. Purified antibodies against a fusion protein comprising a β-lactamase or a fragment thereof and a gp63 protease of L. major.

17. A plasmid corresponding to plasmid pIPS42 shown in figure 1 and deposited at the CNCM on September 26, 1994 under accession number I-1478 and identified as MYC1155.

18. A recombinant strain of BCG expressing a fusion protein including β-lactamase or a portion thereof and a gp63 protease of L. major.

19. A recombinant BCG strain comprising plasmid pIPS42 deposited at the CNCM on September 26, 1994 under accession number I-1478 and identified as MYC1155.
